# EUROPÄISCHE PATENTSCHRIFT

(11) **EP 1 141 271 B1**
(45) Veröffentlichungstag und Bekanntmachung des Hinweises auf die Patenterteilung: **27.04.2005**
(21) Anmeldenummer: 00903509.8
(22) Anmeldetag: 11.01.2000
(51) Int. Cl.: C12N 15/06, C12N 5/20, C07K 19/00, C12N 15/62, C12N 15/79, C12N 5/10, C07K 16/18

(54) **SELEKTION VON MONOKLONALEN ANTIKÖRPERN**
SELECTION OF MONOCLONAL ANTIBODIES
SELECTION D'ANTICORPS MONOCLONAUX

(30) Priorität: 11.01.1999 DE 19900635
(43) Veröffentlichungstag der Anmeldung: 10.10.2001
(73) Patentinhaber: Deutsches Krebsforschungszentrum Stiftung des öffentlichen Rechts, 69120 Heidelberg (DE)
(72) Erfinder: BREITLING, Frank, D-69115 Heidelberg (DE); POUSTKA, Annemarie, D-69120 Heidelberg (DE); MOLDENHAUER, Gerhard, D-69120 Heidelberg (DE)
(74) Vertreter: Schüssler, Andrea, Dr.
(86) Internationale Anmeldenummer: PCT/DE2000/000079
(87) Internationale Veröffentlichungsnummer: WO 2000/042176

(56) Entgegenhaltungen:
- EP-A- 0 028 902
- WO-A-90/00625
- WO-A-97/08186

## Beschreibung

Die vorliegende Erfindung betrifft ein Verfahren zur Selektion von monoklonalen Antikörpern sowie hierfür verwendbare Mittel.

Die Herstellung von monoklonalen Antikörpern beruht auf einem von Köhler und Milstein entwickelten Verfahren. Nach diesem Verfahren werden B-Lymphozyten mit Myelomzellen fusioniert, wodurch Antikörper-produzierende Hybridomzellen erhalten werden. Ein solches Verfahren weist große Nachteile auf. Insbesondere ist es aufwendig Antikörper zu selektionieren, da dies eine getrennte Kultivierung von Hybridomzellen erfordert. Letzteres führt auch dazu, daß nur eine begrenzte Zahl von Hybridomzellen erfaßt und somit auch nicht alle Antikörper selektioniert werden können, was insbesondere nachteilig ist, wenn Antikörper mit höchster Affinität für ein Antigen selektioniert werden sollen.

EP-A-0 028 902 beschreibt ein Verfahren zur Bildung von Hybridzellen, die einen gewünschten Antikörper herstellen.

WO-A-90/00625 beschreibt ein Vor-Screening-Verfahren, in dem Antigene verwendet werden, um bestimmte B-Zellen, die in einer Familie von B-Zellen gefunden werden, auszuwählen.

Der vorliegenden Erfindung liegt somit die Aufgabe zugrunde, ein Mittel bereitzustellen, mit dem monoklonale Antikörper hergestellt werden können, wobei vorstehende Nachteile vermieden werden.

Erfindungsgemäß wird dies durch die Gegenstände in den Patentansprüchen erreicht.

Die vorliegende Erfindung beruht auf den Erkenntnissen des Anmelders, daß monoklonale Antikörper auf der Zelloberfläche von Hybridomzellen mittels eines Antikörper-Bindeproteins präsentiert werden können. Er hat erkannt, daß hiermit monoklonale Antikörper selektioniert werden können, ohne daß Hybridomzellen getrennt kultiviert werden müssen. Auch hat er erkannt, daß die Selektion von monoklonalen Antikörpern sowohl gegenüber einem bestimmten als auch vielen (un)bestimmten Antigenen einer Antigen-Bibliothek erfolgen kann. Ferner hat er erkannt, daß die Selektion von monoklonalen Antikörpern auch hinsichtlich ihrer Affinitätsstärke zu bestimmten Antigenen erfolgen kann.

Erfindungsgemäß werden die Erkenntnisse des Anmelders genutzt, ein Verfahren zur Selektion von monoklonalen Antikörpern bereitzustellen. Ein solches Verfahren umfaßt die Fusionierung von B-Lymphozyten mit Myelomzellen zu Antikörper-produzierenden Hybridomzellen, wobei die Antikörper auf der Zelloberfläche der Hybridomzellen mittels eines Antikörper-Bindeproteins präsentiert werden, und die Bindung der Antikörper an Antigene, wobei die Antikörper-Bindeproteine über die Myelomzellen in die Hybridomzellen oder über sie kodierende Expressionsvektoren in die Hybridomzellen eingefügt werden.

Der Ausdruck "B-Lymphozyten" umfaßt B-Lymphozyten jeglicher Art und Abstammung. Auch können es Vorstufen von B-Lymphozyten sein. Ferner können die B-Lymphozyten von Tieren, wie Mäusen, Ratten, Kaninchen, etc., oder dem Menschen stammen. Desweiteren können die B-Lymphozyten von einem gesunden oder kranken Organismus stammen. Günstig ist es, wenn sie von einem immunisiertem Organismus stammen. Besonders günstig ist es, wenn die B-Lymphozyten für humane Antikörper oder Teile davon kodieren. Handelt es sich um B-Lymphozyten aus Tieren, ist dies erreichbar, indem die Tiere für die humanen Antikörper bzw. Teile davon transgen sind. Die Herstellung solcher Tiere kann durch übliche Verfahren erfolgen, wobei sich anbietet, die Gene für die humanen Antikörper die Teile davon in embryonale Stammzellen einzuführen, aus denen dann die Tiere generiert werden. Die Bereitstellung von B-Lymphozyten und ihren Vorstufen kann durch übliche Verfahren erfolgen.

Der Ausdruck "Myelomzellen" umfaßt Myelomzellen jeglicher Art. und Abstammung. Auch können es Vorläufer von Myelomzellen sein. Ferner können die Myelomzellen von Tieren, wie Mäusen, Ratten, Kaninchen, etc., oder dem Menschen stammen. Bevorzugte Myelomzellen sind Abkömmlinge der Maus-Stämme P3K, P3-X63.Ag8, X63.Ag8.653, NSO/1, Sp2/O-Ag14 und FO, der Ratten-Stämme Y3-Ag1.2.3, YB2/0 und IR9834, und der menschlichen Stämme U266, SK007 und Karpas 707. Die Bereitstellung von Myelomzellen und ihren Vorstufen kann durch übliche Verfahren erfolgen.

Der Ausdruck Antikörper-produzierende Hybridomzellen" umfaßt Zellen, die durch Fusion von B-Lymphozyten und Myelomzellen entstehen und Antikörper produzieren. Es wird auf die Ausführungen hinsichtlich B-Lymphozyten und Myelomzellen entsprechend verwiesen. Hybridomzellen können tierische und/oder menschliche Nukleinsäuren bzw. Proteine aufweisen. Die Kultivierung von Hybridomzellen kann durch übliche Verfahren erfolgen. Ferner kann es günstig sein, wenn die Hybridomzellen Rekombinasen, z.B. Rag1 oder Rag2, und/oder Mutasen (über)exprimieren. Solches kann durch Transfektion der Hybridomzellen mit entsprechenden Expressionsvektoren erreicht werden. Der Fachmann kennt solche Expressionsvektoren.

Der Ausdruck "Fusion von B-Lymphozyten mit Myelomzellen" betrifft jegliches Verfahren, mit dem diese Zellen fusioniert werden können. Günstig ist ein Verfahren, bei dem die Zellen über Polyethylenglykol fusioniert werden. Es wird auf die Beispiele verwiesen.

Der Ausdruck "Bindung der Antikörper an Antigene" betrifft jegliches Verfahren, mit dem die auf der Zelloberfläche der Hybridomzellen exprimierten Antikörper an Antigene binden können. Die Antigene können an Trägern, z.B. Magnetobeads, gebunden sein. Ferner können sie markiert, z.B. fluoreszenzmarkiert, sein. Als Fluoreszenzmarker bieten sich z.B. FITC, TRITC, Cy3, Cy5, Cy5.5, Cy7 und Phycoerythrin an. Desweiteren können die Antigene an Biotin gekoppelt sein. Gebundene Antigene können dann durch übliche Verfahren, z.B. FACS-Analyse, nachgewiesen werden, wodurch auch die entsprechenden Antikörper detektiert werden. Es wird auf die Beispiele verwiesen.

Der Ausdruck "Antikörper-Bindeprotein" umfaßt jegliches Protein, das einen Antikörper binden und an der Zelloberfläche von Hybridomzellen präsentieren kann. Insbesondere kann das Protein ein Signalpeptid, eine von der Spezifität des Antikörpers unabhängige Antikörper-Bindestelle und einen Membrananker aufweisen. Beispiele für ein solches Protein sind natürliche Fc-Bindeproteine, wie CD16, CD32 und CD64. Ferner kann das Protein eine Kombination aus einem Signalpeptid, einer Antikörper-Bindestelle und einem Membrananker aufweisen, die in der Natur nicht vorkommt. Eine solche Kombination kann Teile natürlicher Fc-Bindeproteine umfassen. Ferner kann sie als Signalpeptid ein solches einer Maus-Ig-Kappa-Kette oder eines Maus-MHC-Klasse I k(k)-Moleküls, als Membrananker eine Transmembran-Domäne von PDGRF oder CD52 und als Antikörper-Bindestelle eine Antigen-Bindungsdomäne eines bakteriellen Proteins, wie Protein A, Protein G, Protein L oder Protein LG, aufweisen. Günstig kann es sein, wenn die Kombination mehrere Signalpeptide, Antikörper-Bindestellen und/oder Membrananker aufweist. Besonders günstig kann es sein, wenn das Antikörper-Bindeprotein, insbesondere die Antikörper-Bindungsdomäne der bakteriellen Proteine Codons aufweist, die für die Expression in Säugetierzellen optimiert sind. Der Fachmann weiß, um welche Codons es sich hier handelt.

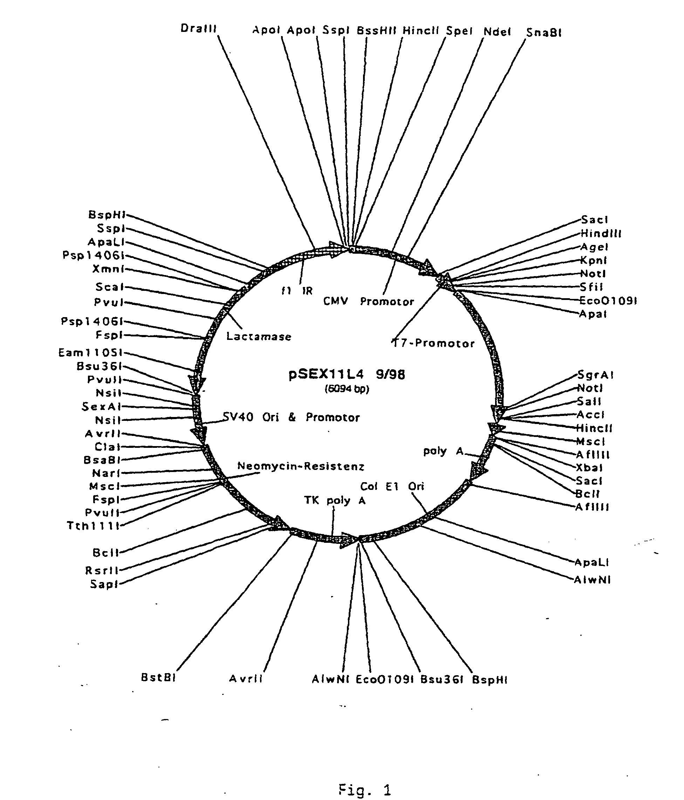

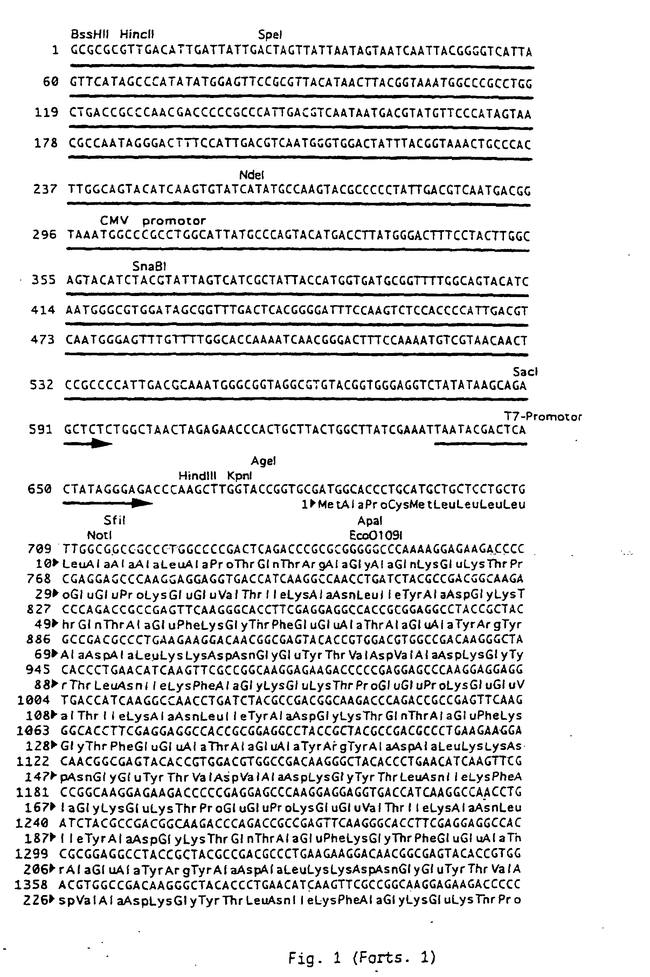

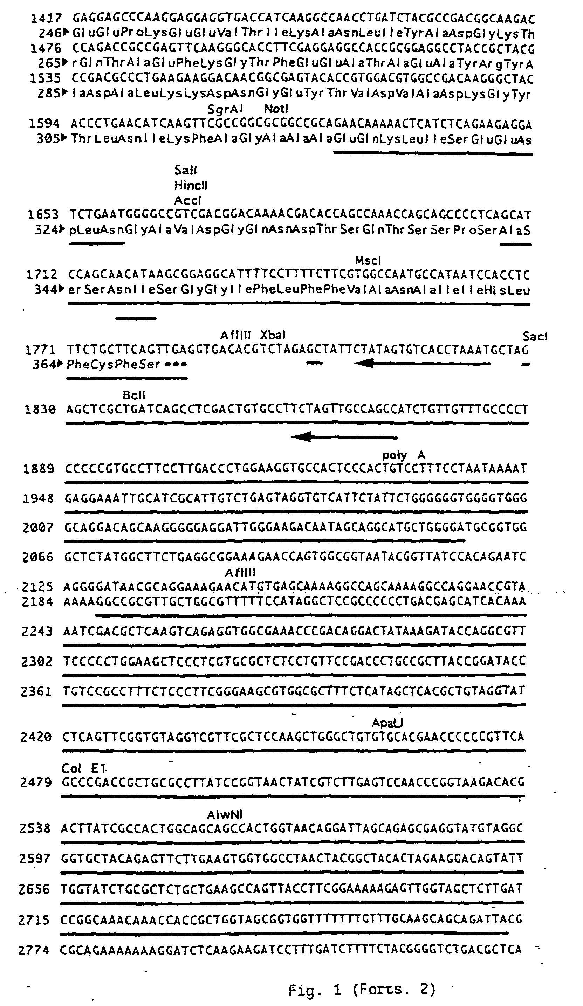

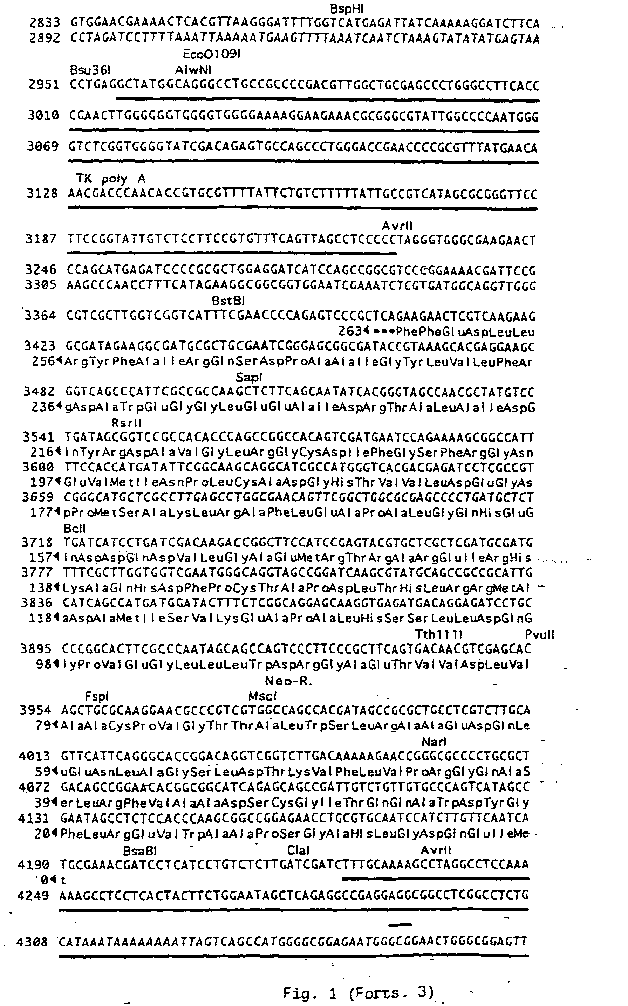

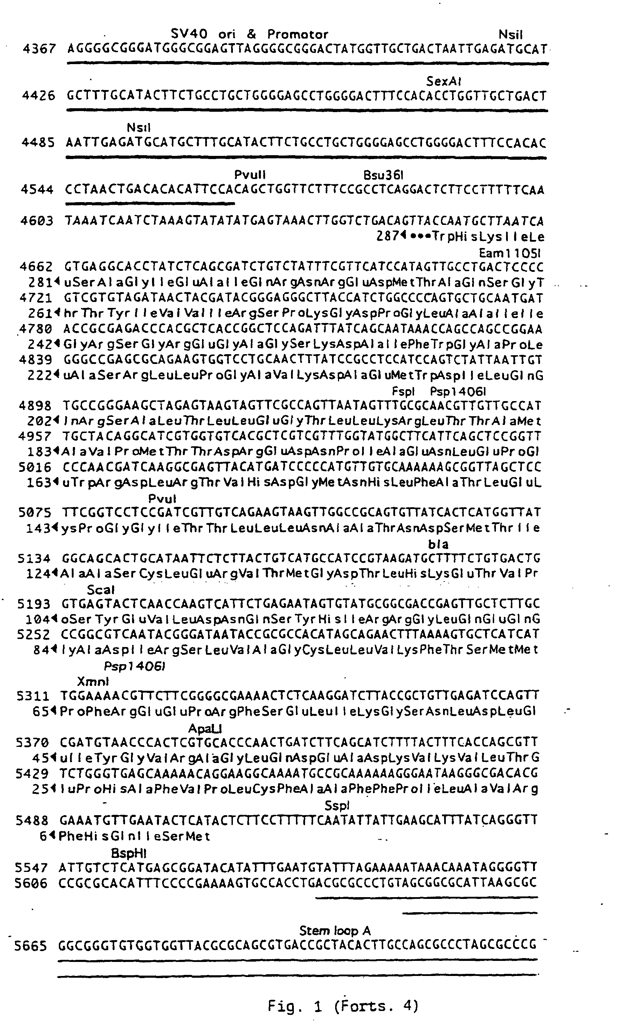

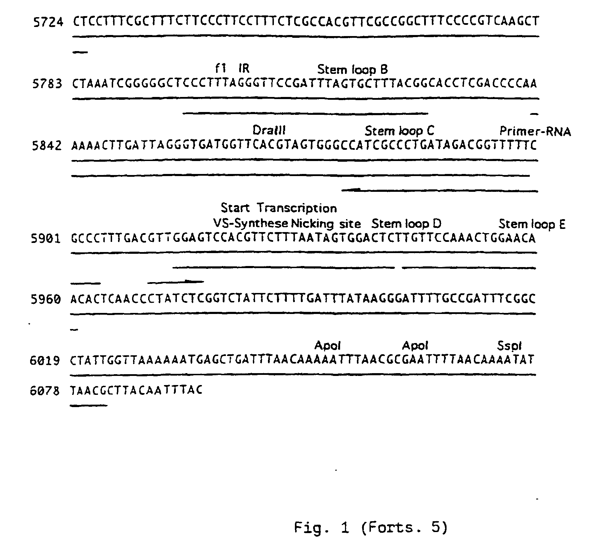

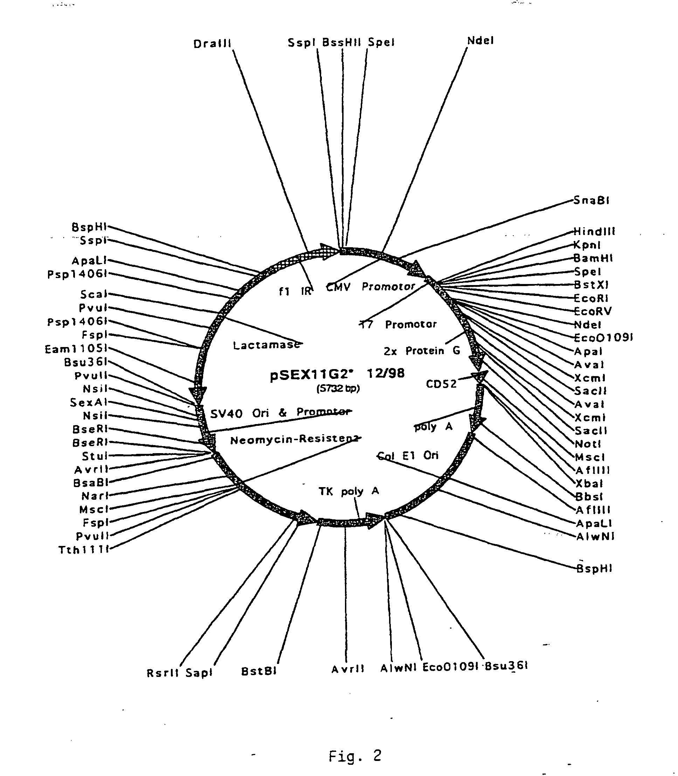

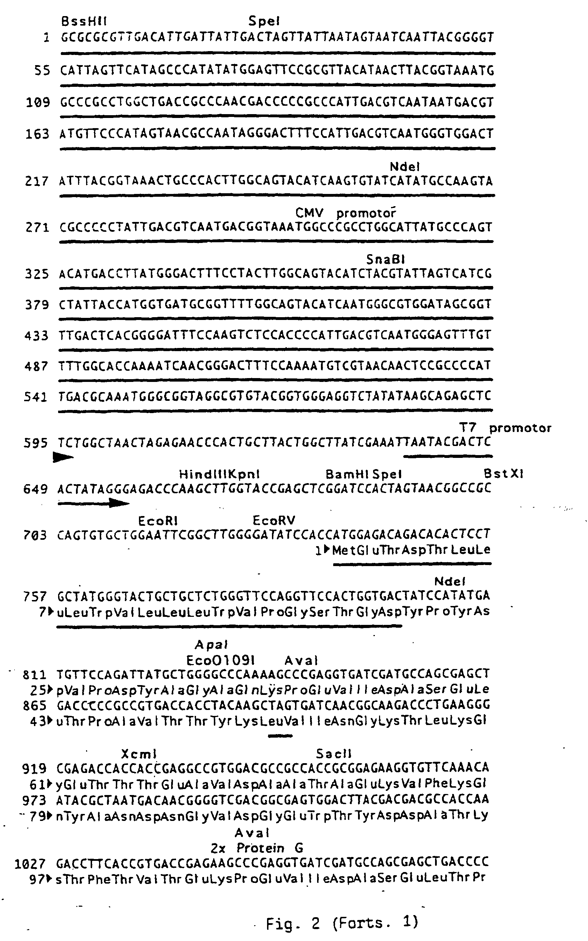

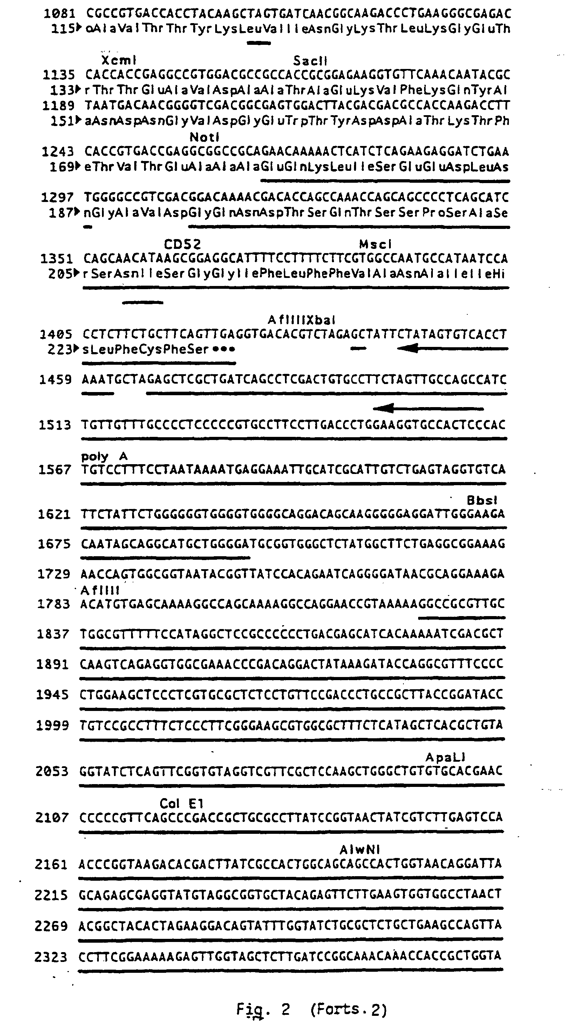

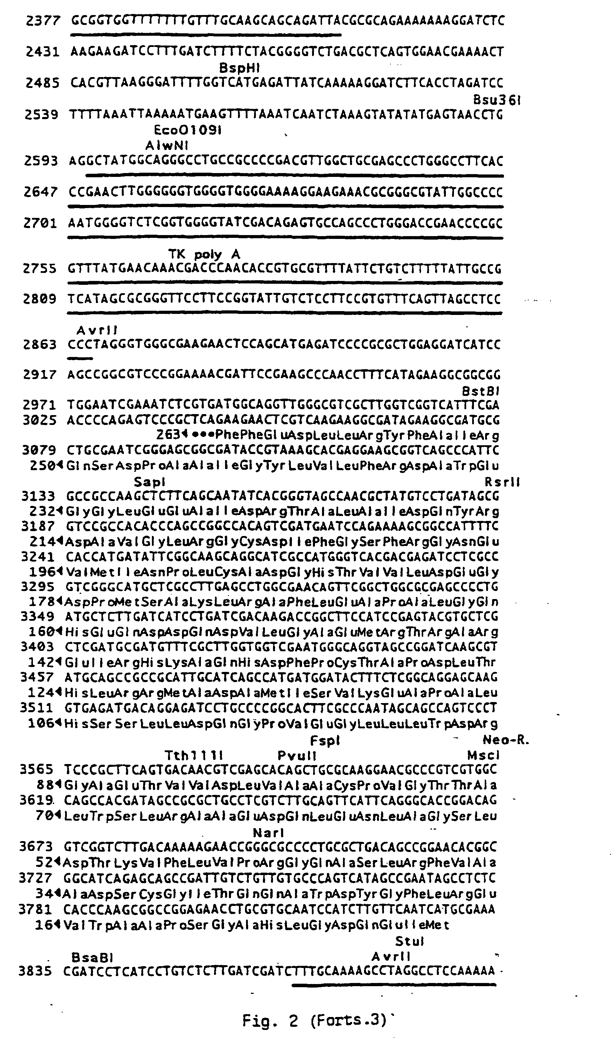

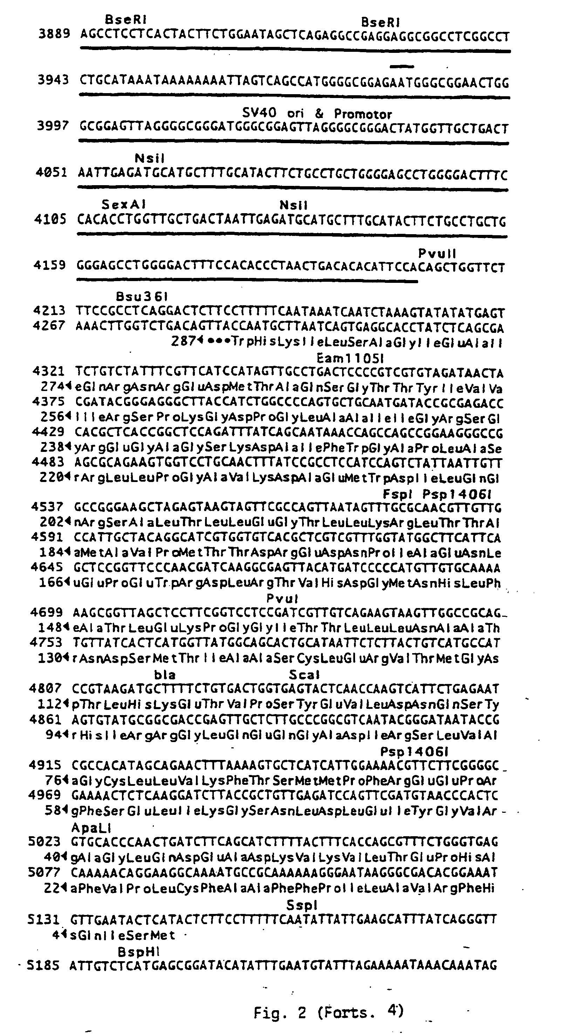

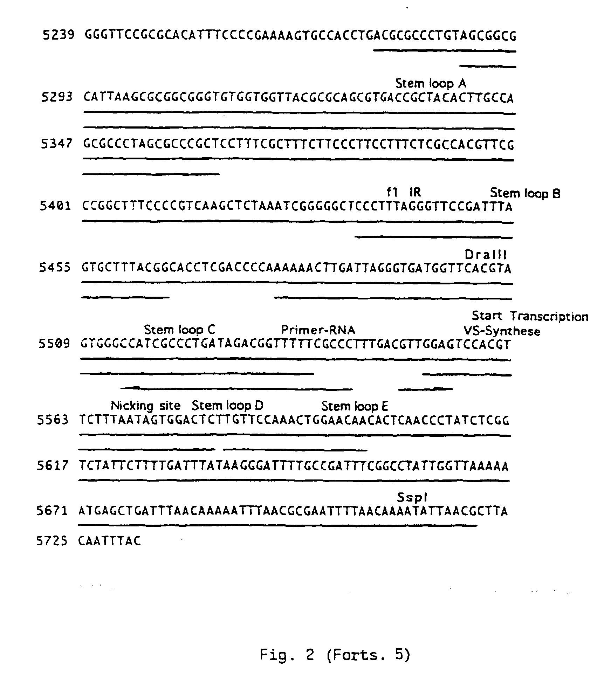

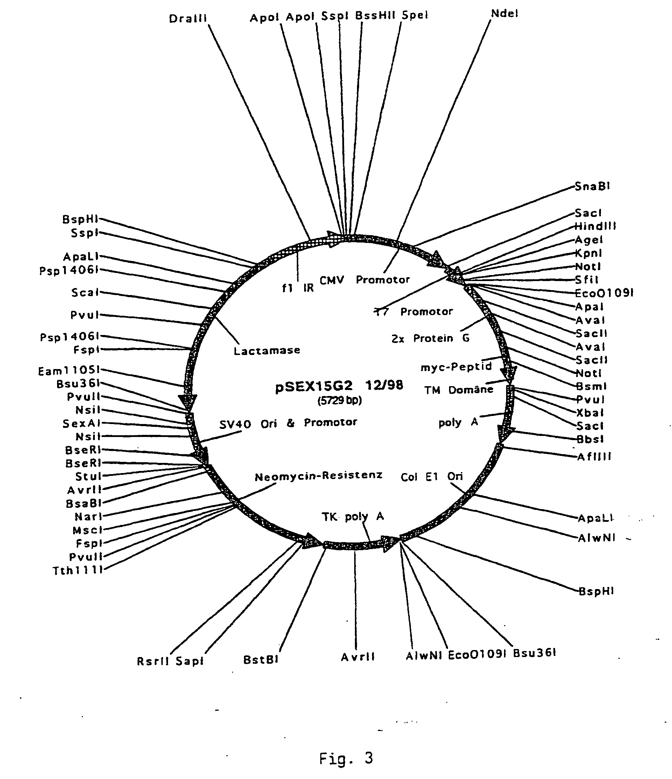

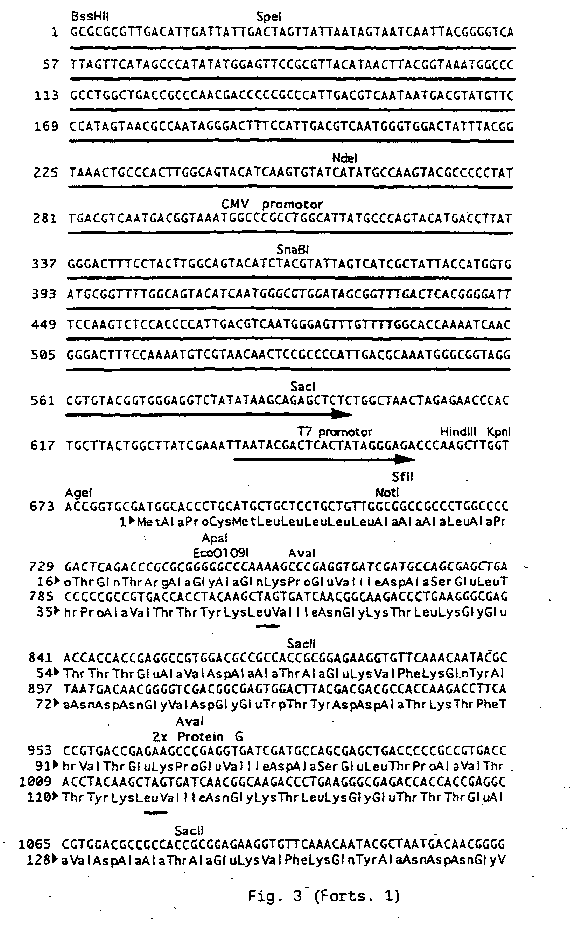

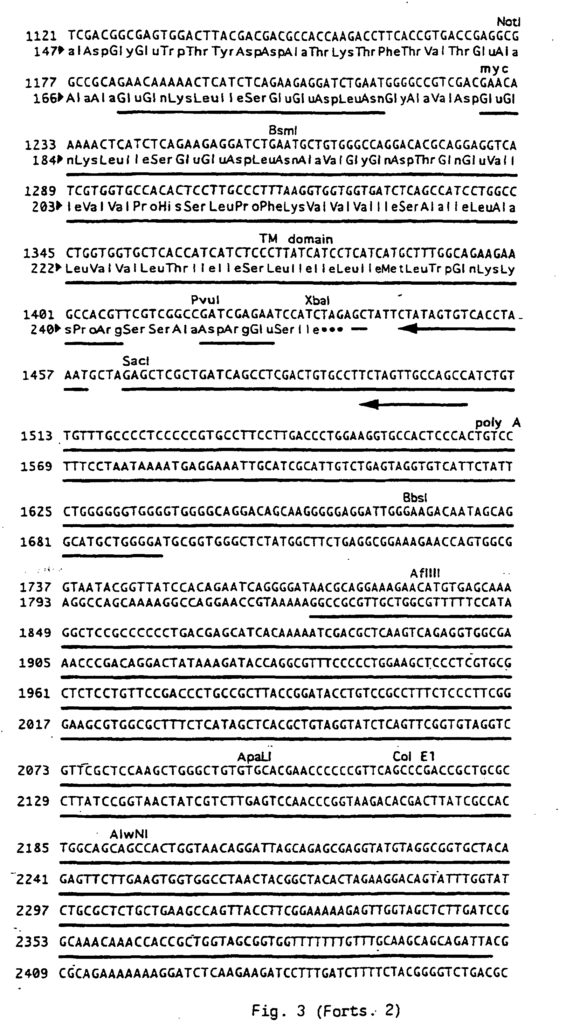

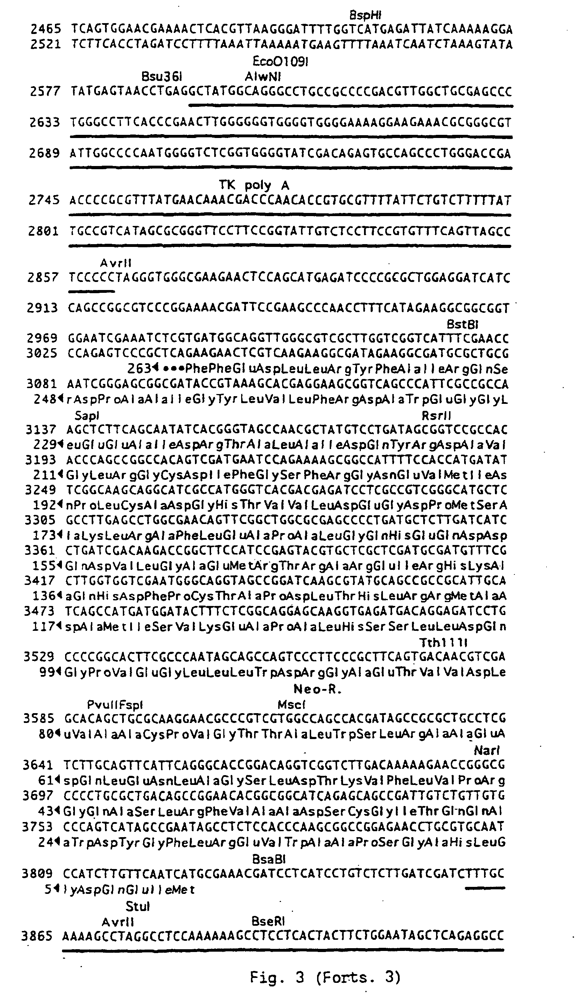

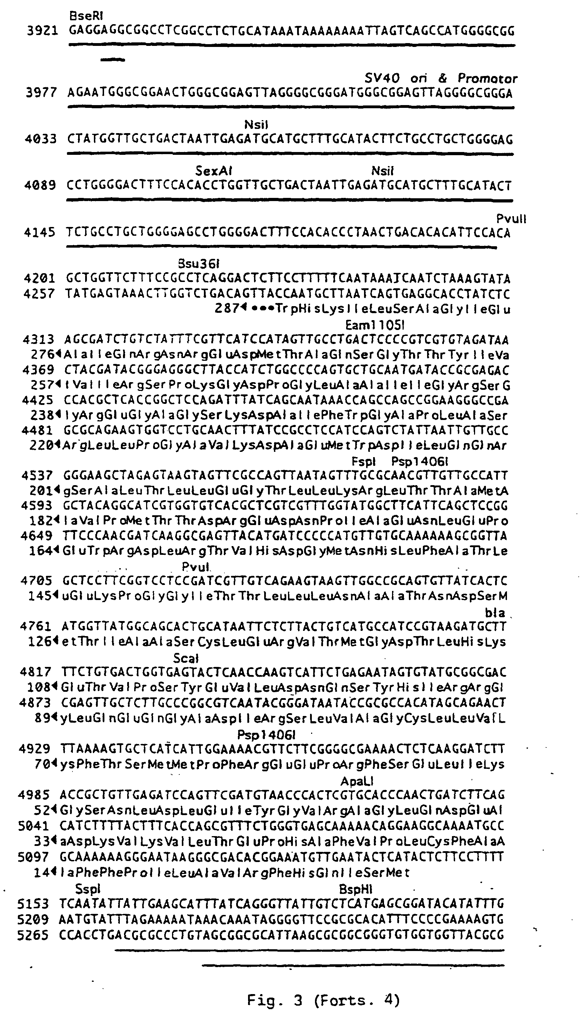

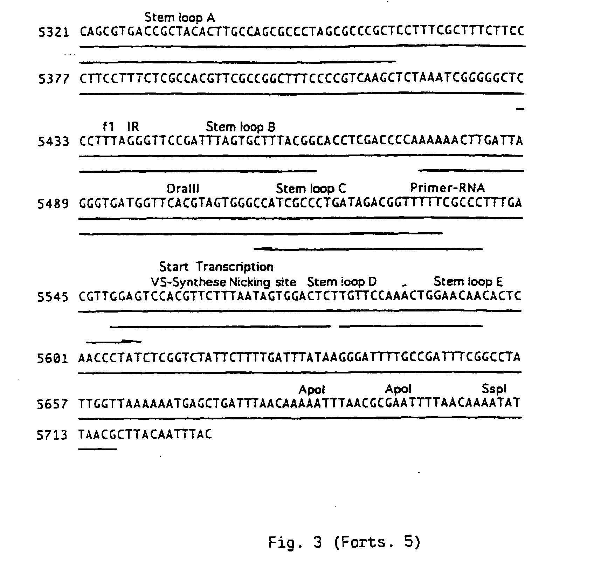

Bevorzugte Antikörper-Bindeproteine sind in den Figuren 1-3 angegeben. Das Antikörper-Bindeprotein von Fig. 1 umfaßt das Signalpeptid eines Maus-MHC-Klasse I k(k)-Moleküls, vier Antikörper-Bindungsdomänen des Proteins L und die Transmembran-Domäne von CD52. Die DNA- und Aminosäuresequenzen des Antikörper-Bindeproteins sind zwischen den Nukleotid-Nummern 682-1782 angegeben. Das Antikörper-Bindeprotein von Fig. 2 umfaßt das Signalpeptid einer Maus-Ig-Kappa-Kette, zwei Antikörper-Bindestellen des Proteins G und die Transmembran-Domäne von CD52. Die DNA- und Aminosäuresequenzen des Antikörper-Bindeproteins sind zwischen den Nukleotid-Nummern 737-1420 angegeben. Das Antikörper-Bindeprotein von Fig. 3 umfaßt das Signalpeptid des . Maus-MHC-Klasse I k(k)-Moleküls, zwei Antikörper-Bindestellen des Proteins G und die Transmembran-Domäne von PDGFR. Die DNAund Aminosäuresequenzen des Antikörper-Bindeproteins sind zwischen den Nukleotid-Nummern 682-1431 angegeben. Die Antikörper-Bindestellen aller drei Antikörper-Bindeproteine weisen auf DNA-Ebene Codons auf, die für die Expression in Säugetierzellen optimiert sind.

Ein weiterer Gegenstand der vorliegenden Erfindung ist eine Nukleinsäure, die für ein vorstehendes Antikörper-Bindeprotein kodiert. Die Nukleinsäure ist eine DNA, die folgendes umfaßt:
(a) Die DNA eines Antikörper-Bindeproteins der Figuren 1, 2 bzw. 3, oder
(b) eine mit der DNA von (a) über den degenerierten genetischen Code verwandte DNA.

Eine erfindungsgemäße DNA kann als solche oder in Kombination mit jeglicher anderen DNA vorliegen. Insbesondere kann eine erfindungsgemäße, für ein Antikörper-Bindeprotein kodierende DNA in einem Expressionsvektor vorliegen. Beispiele solcher sind dem Fachmann bekannt. Im Falle eines Expressionsvektors für E. coli sind dies z.B. pGEMEX, pUC-Derivate, pGEX-2T, pET3b und pQE-8. Für die Expression in Hefe sind z.B. pY100 und Ycpad1 zu nennen, während für die Expression in tierischen Zellen z.B. pKCR, pEFBOS, pCDM8 und pCEV4 anzugeben sind. Für die Expression in Insektenzellen eignet sich besonders der Bacculovirus-Expressionsvektor pAcSGHisNT-A.

Der Fachmann weiß, in welcher Weise die erfindungsgemäße DNA in einen Expressionsvektor inseriert werden muß. Ihm ist auch bekannt, daß diese DNA in Verbindung mit einer für ein anderes Protein bzw. Peptid kodierenden DNA inseriert werden kann, so daß die erfindungsgemäße DNA in Form eines Fusionsproteins exprimiert werden kann.

Bevorzugte Expressionsvektoren, die eine erfindungsgemäße DNA enthalten, sind in den Figuren 1-3 angegeben. Es handelt sich um die Expressionsvektoren pSEX11L4, pSEX11G2* und pSEX15G2. Diese wurden bei der DSMZ (Deutsche Sammelung für Mikroorganismen und Zellkulturen) am 14. Dezember 1998 hinterlegt. Im einzelnen wurde pSEX11L4 unter DSM 12580, pSEX11G2^{*} unter DSM 12581 und pSEX15G2 unter DSM 12582 hinterlegt.

Der Fachmann kennt geeignete Zellen, um die erfindungsgemäße, in einem Expressionsvektor vorliegende DNA zu exprimieren. Beispiele solcher Zellen umfassen die E.coli-Stämme XL-1 Blue, Top 10 F, HB101, DH5alpha, x1776, JM101, JM 109, BL21 und SG 13009, die Hefe-Stämme Saccharomyces cerevisiae und Pichia pastoris, die tierischen Zellen L, NIH 3T3, FM3A, CHO, COS, Vero, HeLa, Myelom- und Hybridomzellen sowie die Insektenzellen sf9.

Desweiteren kennt der Fachmann Bedingungen, transformierte bzw. transfizierte Zellen zu kultivieren. Auch sind ihm Verfahren bekannt, das durch die erfindungsgemäße DNA exprimierte Protein bzw. Fusionsprotein zu isolieren und zu reinigen.

Die vorliegende Erfindung zeichnet sich dadurch aus, daß von Hybridomzellen produzierte Antikörper auf der Zelloberfläche der Hybridomzellen präsentiert werden. Dies erfolgt über ein Antikörper-Bindeprotein. Ein solches wird über die zur Herstellung der Hybridomzellen verwendeten Myelomzellen in die Hybridomzellen eingeführt oder über einen es kodierenden Expressionsvektor.

Mit der vorliegenden Erfindung ist es möglich, Antikörper zu selektionieren. Dies kann ohne großen Aufwand erfolgen, da Hybridomzellen nicht getrennt kultiviert werden müssen. Vielmehr können komplexe Gemische von Hybridomzellen unmittelbar zur Selektion von Antikörpern verwendet werden. Ferner können Antikörper hinsichtlich ihrer Affinitätsstärke zu bestimmten Antigenen selektioniert werden. Desweiteren eignet sich die vorliegende Erfindung Antikörper von Hybridomzell-Bibliotheken nicht nur gegenüber einem bestimmten Antigen, sondern auch gegenüber vielen (un)bestimmten Antigenen von Antigen-Bibliotheken zu selektionieren.

Somit liefert die vorliegende Erfindung ein Mittel, mit dem u.a. die großen Zeit- und Kosten-Probleme vermieden werden können, die bei der Selektion von monoklonalen Antikörpern bisher auftraten.

### Kurze Beschreibung der Zeichnungen

- Fig. 1: zeigt den erfindungsgemäßen Expressionsvektor pSEX11L4 (Fig. 1(A)), der für ein Antikörper-Bindeprotein kodiert (Fig. 1(B)). Es wird auf vorstehende Ausführungen verwiesen.
- Fig. 2: zeigt den erfindungsgemäßen Expressionsvektor pSEX11G2* (Fig. 2(A)), der für ein Antikörper-Bindeprotein kodiert (Fig. 2(B)). Es wird auf vorstehende Ausführungen verwiesen.
- Fig. 3: zeigt den erfindungsgemäßen Expressionsvektor pSEX15G2 (Fig. 3(A)), der für ein Antikörper-Bindeprotein kodiert (Fig. 3(B)). Es wird auf vorstehende Ausführungen verwiesen.

Die Erfindung wird durch die nachfolgenden Beispiele erläutert.

### Beispiel 1: Herstellung von Myelomzellen, die ein Antikörper-Bindeprotein auf ihrer Zelloberfläche exprimieren.

### (A) Transiente Expression

Es werden Zellen der Myelomzellinie X63-Ag8.653 verwendet. Diese Zellen (10⁷) werden mit 20-40 µg des erfindungsgemäßen Expressionsvektors
pSEX11G2* (vgl. Fig. 2) transfiziert. Als Transfektionstechnik wird eine Elektroporation durchgeführt, die zwei Pulse zu 2 ms bei 500 V umfaßt. Die Zellen werden 48 h in RPMI-Medium, das 10 % FCS enthält, bei 37°C und 5-7,5 % Co₂ inkubiert. Danach werden die Zellen mit kaltem DPBS + 0.1 % Na-Azid gewaschen, bevor sie 45 min bei 0°C mit DPBS + 0.1 % Na-Azid plus 25 µg/ml Ziege anti-Kalb Antikörper (FITC markiert; GAB-FITC, Dianova) inkubiert werden. Nach Waschen mit DPBS + 0.1 % Na-Azid werden die Zellen in DPBS + 0.1 % Na-Azid + 1 µg/ml Propidium-Jodid inkubiert und nach Anregung mit Blaulicht einer FACS-Analyse unterzogen.

Es zeigt sich, daß die transfizierten Myelomzellen eine grüne Fluoreszenz aufweisen, die durch die transiente Expression eines Antikörper-Bindeproteins auf der Zelloberfläche der Myelomzellen bedingt ist.

### (B) Stabile Expression

Die unter (A) erhaltenen-Myelomzellen werden einer 14-24 tägigen G418-Selektion unterzogen, bevor sie, wie unter (A) beschrieben, mit GAB-FITC inkubiert und einer FACS-Analyse unterzogen werden. Myelomzellen, die eine starke grüne Fluoreszenz aufweisen, werden weiteren G418-Selektionsrunden unterzogen.

Es wird die Myelomzelline X63-Ag8.653.3 erhalten, die stabil ein Antikörper-Bindeprotein auf ihrer Zelloberfläche exprimiert.

### Beispiel 2: Herstellung von Hybridomzellen, die auf ihrer Zelloberfläche Antikörper mittels eines Antikörper-Bindeproteins exprimieren.

**(A)**
   Es werden 10 Balb/c-Mäuse subkutan mit je 100 µg abgetöteten Helicobacter pylori Bakterien in komplettem Freund' sehen Adjuvans, das abgetötete Mycobacter tuberculosis Bakterien enthält, immunisiert. Nach 4 bzw. 7 Wochen erfolgt jeweils eine intraperitoneale Booster-Injektion mit 100µg abgetöteter Helicobacter pylori-/Mycobacter tuberculosis-Bakterien. Den Mäusen werden vor jeder Immunisierung bzw. nach der letzten Immunisierung jeweils 100 µl Blutserum entnommen und im Westernblot wird die Antigenspezifische Immunantwort der Maus überprüft. Als Antigen wird ein Aufschluß von bakteriellem Gesamtprotein von Helicobacter pylori bzw. Mycobacter tuberculosis verwendet. Der Nachweis gebundener Maus-Antikörper wird durch einen Peroxidase-konjugierten Ziege anti-Maus-Antikörper (Dianova) geführt. Mäusen mit einer deutlichen Antigen-spezifischen Immunantwort wird die Milz entnommen und die Lymphozyten werden mit Zellen der Myelomzelline X63-Ag8.653.3 von Beispiel 1 (B) fusioniert. Die Fusion erfolgt durch Polyethylenglykol (vgl. Goding, J.W., Cell Biology, Biochemistry and Irnmunology, 3. Auflage, (1996), Verlag Accademic Press Limited, 24-28). Es werden Hybridomzellen erhalten. Diese werden 10-12 Tage in HAT-Medium bei 37°C inkubiert. Es wird die Hybridomzell-Bibliothek 2A erhalten.
   Es werden Hexapeptide mit N-terminalem Biotin synthetisiert. Die Peptide entsprechen den 6C-terminalen Aminosäuren von 101 bzw. 118 Genprodukten von Helicobacter pylori bzw. Mycobacter tuberculosis. Ferner werden 10³ Zellen der Hybridomzell-Bibliothek 2A mit kaltem DPBS + 0,1 % Na-Azid gewaschen und 45 min bei 0°C mit DPBS + 0,1 % Na-Azid + 10µg/ml der vorstehenden Biotin-markierten Peptide inkubiert. Die Zellen werden mit kaltem DPBS + 0,1 % Na-Azid gewaschen und 45 min bei 0°C mit 10µg/ml Steptavidin-FITC inkubiert. Nach Waschen mit DPBS + 0,1 % Na-Azid werden die Zellen in DPBS + 0,1 % Na-Azid + 1µg/ml Propidium-Jodid inkubiert und nach Anregung mit Blaulicht einer FACS-Analyse unterzogen.
   Es zeigt sich, daß die Hybridomzellen eine grüne Fluoreszenz aufweisen. Diese Fluoreszenz ist durch die Expression von Antikörpern auf der Zelloberfläche der Hybridomzellen bedingt. Weiterführende Untersuchungen zeigen, daß die Antikörper eine anti-Helicobacter pylori- bzw. Mycobacter tuberculosis-Aktivität aufweisen.
**(B)**
   Es werden Zellen der Hybridomzellinie U98/6, die einen Mausanti-Urokinase-Antikörper produzieren, verwendet. Diese Zellen (10⁷) werden mit 20-40µg
   des erfindungsgemäßen Expressionsvektors pSEX11G2* (vgl. Fig. 2) transfiziert. Als Transfektionstechnik wird eine Elektroporation durchgeführt, die zwei Pulse zu 2 ms bei 400 V umfaßt. Die Zellen werden 48 h in inkomplettem AIM V-Medium bei 37°C und 5-7,5 % Co₂ inkubiert. Danach werden die Zellen mit kaltem DPBS + 0.1 % Na-Azid gewaschen, bevor sie 45 min bei 0°C mit DPBS + 0.1 % Na-Azid + 10 µg/ml Urokinase-Biotin inkubiert werden. Nach Waschen mit DPBS + 0.1 % Na-Azid werden die Zellen in DPBS + 0.1 % Na-Azid + 10 µg/ml Streptavidin-FITC inkubiert und nach Anregung mit Blaulicht einer FACS-Analyse unterzogen.

Es zeigt sich, daß die transfizierten Hybridomzellen eine grüne Fluoreszenz aufweisen. Diese Fluoreszenz ist durch die Expression von Antikörpern auf der Zelloberfläche der Hybridomzellen bedingt. Weiterführende Untersuchungen zeigen, daß die Antikörper eine anti-Urokinase-Aktivität aufweisen.

Die erhaltenen Hybridomzellen werden einer 14-24 tägigen G418-Selektion unterzogen, bevor sie erneut, wie vorstehend beschrieben mit Urokinase-Biotin und Streptavidin-FICS inkubiert und einer FACS-Analyse unterzogen werden. Hybridomzellen, die eine starke grüne Fluoreszenz aufweisen, werden weiteren G418-Selektionsrunden unterzogen.

Es wird die Hybridomzelline U98/6.3.3 erhalten, die stabil Antikörper auf ihrer Zelloberfläche exprimiert.

### Beispiel 3: Selektion von monoklonalen Antikörpern, die mittels eines Antikörper-Bindeproteins auf der Zelloberfläche von Hybridomzellen exprimiert werden.

10³ Zellen der Hybridomzellinie U98/6.3.3 von Beispiel 2 (B) werden mit 10⁷ Zellen der Hybridomzellinie DOB.L1.3 gemischt. Letztere Hybridomzellinie produziert einen den C-Terminus der humanen HLA-DO-β-Kette erkennenden Antikörper. Dieser wird mittels des gleichen Antikörper-Bindeproteins wie in der Hybridomzellinie U98/6.3.3 von Beispiel 2 (B) auf der Zelloberfläche exprimiert. Das Zellgemisch wird mit kaltem DPBS + 0,1 % Na-Azid gewaschen und 45 min bei 0°C mit DPBS + 0,1 % Na-Azid + 10 µg/ml Urokinase-Biotin inkubiert. Nach Waschen mit DPBS + 0,1 % Na-Azid wird das Zellgemisch in DPBS + 0,1 % Na-Azid + 10 µg/ml Streptavidin-FITC inkubiert und nach Anregung mit Blaulicht in einen FACS-Sorter gegeben.

Es werden Hybridomzellen mit grüner Fluoreszenz selektioniert. In weiterführenden Untersuchungen zeigen diese eine anti-Urokinase-Aktivität. Es werden die Hybridomzellinien U98/6.3.3 S1-S50 erhalten.

### Beispiel 4: Herstellung und Reiniguag eines erfindungsgemäßen Antikörper-Bindeproteins

**(A)**
   Die DNA von Fig. 1 zwischen den Nukleotid-Nummern 682-1782 wird mit BAMHI-Linkern versehen, mit BamHI nachgespalten und in den mit BamHI gespaltenen Expressionsvektors pQE-8 (Qiagen) inseriert. Es wird das Expressionsplasmid pQE-8/Antikörper-Bindeprotein erhalten. Ein solches kodiert für ein Fusionsprotein aus 6 Histidin-Resten (N-Terminuspartner) und dem erfindungsgemäßen Antikörper-Bindeprotein von Fig. 1 (C-Terminuspartner). pQE-8/Antikörper-Bindeprotein wird zur Transformation von E.coli SG 13009(vgl. Gottesman, S. et al., J. Bacteriol. 148, (1981), 265-273) verwendet. Die Bakterien werden in einem LB-Medium mit 100µg/ml Ampicillin und 25µg/ml Kanamycin kultiviert und 4 h mit 60µM Isopropyl-β-D-Thiogalactopyranosid (IPTG) induziert. Durch Zugabe von 6 M Guanidinhydrochlorid wird eine Lyse der Bakterien erreicht, anschließend wird mit dem Lysat eine Chromatographie (Ni-NTA-Resin) in Gegenwart von 8 M Harnstoff entsprechend der Angaben des Herstellers (Qiagen) des Chromatographie-Materials durchgeführt. Das gebundene Fusionsprotein wird in einem Puffer mit pH 3,5 eluiert. Nach seiner Neutralisierung wird das Fusionsprotein einer 18 % SDS-Polyacrylamid-Gelelektrophorese unterworfen und mit Coomassie-Blau angefärbt (vgl. Thomas, J.O. und Kornberg, R.D., J.Mol.Biol. 149 (1975), 709-733).
   Es zeigt sich, daß ein erfindungsgemäßes Antikörper-Bindeprotein (Fusionsprotein) in hochreiner Form hergestellt werden kann.
**(B)**
   10⁸ Zellen der in Beispiel 1 (B) erhaltenen Myelomzelllinie X63-Ag8.653.3 werden mit PBS gewaschen, in PBS + 1 % Tween 20 aufgenommen und auf Eis inkubiert. Partikuläre Zellbestandteile werden durch Zentrifugation bei
   30.000g abgetrennt und der Überstand wird auf eine IgG Sepharose Säule (IgG Sepharose 6 Fast Flow Lab Pack von Pharmacia) gegeben. Ungebundene Bestandteile werden durch Waschen entfernt und das erfindungsgemäße Antikörper-Bindeprotein wird in saurem pH eluiert.

Nach seiner Neutralisierung wird das Antikörper-Bindeprotein einer 18 % SDS-Polyacrylamid-Gelelectrophorese unterworfen und mit Coomassie-Blau angefärbt (vgl. vorstehend).

Es zeigte sich, daß ein erfindungsgemäßes Antikörper-Bindeprotein (Fusionsprotein) in hochreiner Form hergestellt werden kann.

### Beispiel 5: Herstellung und Nachweis eines erfindungsgemäßen Antikörpers

Ein erfindungsgemäßes Fusionsprotein von Beispiel 4 wird einer 18 % SDS-Polyacrylamid-Gelelektrophorese unterzogen. Nach Anfärbung des Gels mit 4 M Natriumacetat wird eine ca. 41 kD Bande aus dem Gel herausgeschnitten und in Phosphat gepufferter Kochsalzlösung inkubiert. Gel-Stücke werden sedimentiert, bevor die Proteinkonzentration des Überstandes durch eine SDS-Polyacrylamid-Gelelektrophorese, der eine Coomassie-Blau-Färbung folgt, bestimmt wird. Mit dem Gel-gereinigten Fusionsprotein werden Tiere wie folgt immunisiert:

### Immunisierungsprotokoll für polyklonale Antikörper im Kaninchen

Pro Immunisierung werden 35 µg gereinigtes Fusionsprotein in 0,7 ml PBS und 0,7 ml komplettem bzw. inkomplettem Freund's Adjuvans eingesetzt.
- Tag O:: 1. Immunisierung (komplettes Freund's Adjuvans)
- Tag 14:: 2. Immunisierung (inkomplettes Freund's Adjuvans; icFA)
- Tag 28:: 3. Immunisierung (icFA)
- Tag 56:: 4. Immunisierung (icFA)
- Tag 80:: Ausbluten

Das Serum des Kaninchens wird im Immunoblot getestet. Hierzu wird ein erfindungsgemäßes Fusionsprotein von Beispiel 4 einer SDS-Polyacrylamid-Gelelektrophorese unterzogen und auf ein - Nitrocellulosefilter übertragen (vgl. Khyse-Andersen, J., J. Biochem. Biophys. Meth. 10, (1984), 203-209). Die Western Blot-Analyse wurde wie in Bock, C.-T. et al., Virus Genes 8, (1994), 215-229, beschrieben, durchgeführt. Hierzu wird das Nitrocellulosefilter eine Stunde bei 37°C mit einem ersten Antikörper inkubiert. Dieser Antikörper ist das Serum des Kaninchens (1:10000 in PBS). Nach mehreren Waschschritten mit PBS wird das Nitrocellulosefilter mit einem zweiten Antikörper inkubiert. Dieser Antikörper ist ein mit alkalischer Phosphatase gekoppelter monoklonaler Ziege Anti-Kaninchen-IgG-Antikörper (Dianova) (1:5000) in PBS. Nach 30-minütiger Inkubation bei 37°C folgen mehrere Waschschritte mit PBS und anschließend die alkalische Phosphatase-Nachweisreaktion mit Entwicklerlösung (36µM 5' Bromo-4-chloro-3-indolylphosphat, 400µM Nitroblau-tetrazolium, 100mM Tris-HCl, pH 9.5, 100 mM NaCl, 5 mM MgCl₂) bei Raumtemperatur, bis Banden sichtbar werden.

Es zeigt sich, daß erfindungsgemäße, polyklonale Antikörper hergestellt werden können.

### Immunisierungsprotokoll für polyklonale Antikörper im Huhn

Pro Immunisierung werden 40µg gereinigtes Fusionsprotein in 0,8 ml PBS und 0,8 ml komplettem bzw. inkomplettem Freund's Adjuvans eingesetzt.
- Tag O.: 1. Immunisierung (komplettes Freund's Adjuvans)
- Tag 28:: 2. Immunisierung (inkomplettes Freund's Adjuvans; icFA)
- Tag 50:: 3. Immunisierung (icFA)

Aus Eigelb werden Antikörper extrahiert und im Western Blot getestet. Es werden erfindungsgemäße, polyklonale Antikörper nachgewiesen.

### Immunisierungsprotokoll für monoklonale Antikörper der Maus

Pro Immunisierung werden 12µg gereinigtes Fusionsprotein in 0,25 ml PBS und 0,25 ml komplettem bzw. inkomplettem Freund's Adjuvans eingesetzt; bei der 4. Immunisierung ist das Fusionsprotein in 0,5 ml (ohne Adjuvans) gelöst.
- Tag O.: 1. Immunisierung (komplettes Freund's Adjuvans)
- Tag 28:: 2. Immunisierung (inkomplettes Freund's Adjuvans; icFA)
- Tag 56:: 3. Immunisierung (icFA)
- Tag 84:: 4. Immunisierung (PBS)
- Tag 87:: Fusion

Überstände von Hybridomen werden im Western Blot getestet. Erfindungsgemäße, monoklonale Antikörper werden nachgewiesen.

## Patentansprüche

1. Verfahren zur Selektion von monoklonalen Antikörpern, umfassend die Fusion von B-Lymphozyten mit Myelomzellen zu Antikörper-produzierenden Hybridomzellen, wobei die Antikörper auf der Zelloberfläche der Hybridomzellen mittels eines Antikörper-Bindeproteins präsentiert werden, und die Bindung der Antikörper an Antigene, wobei die Antikörper-Bindeproteine über die Myelomzellen in die Hybridomzellen oder über sie kodierende Expressionsvektoren in die Hybridomzellen eingefügt werden.

2. Verfahren nach Anspruch 1, wobei das Antikörper-Bindeprotein ein Signalpeptid, eine von der Spezifität des Antikörpers unabhängige Antikörper-Bindestelle und einen Membrananker umfaßt.

3. Verfahren nach Anspruch 2, wobei das Antikörper-Bindeprotein ein Fc-Bindeprotein oder Teile davon umfaßt.

4. Verfahren nach Anspruch 2, wobei das Antikörper-Bindeprotein eine Kombination aus Fc-Bindeproteinen oder Teilen davon umfaßt.

5. Verfahren nach Anspruch 3 oder 4, wobei das Fc-Bindeprotein CD16, CD32 oder CD64 ist.

6. Verfahren nach einem der Ansprüche 2 - 5, wobei das Antikörper-Bindeprotein eine Antikörper-Bindungsdomäne der Proteine A, G, L oder LG umfaßt.

7. Verfahren nach Anspruch 2, wobei das Antikörper-Bindeprotein eine Kombination aus dem Signalpeptid einer Maus-Ig-Kappa-Kette oder eines Maus-MHC-Klasse I k(k)-Moleküls, einer Antikörper-Bindestelle der Proteine A, G, L oder LG und der Transmembran-Domäne von PDGFR oder CD52 umfaßt.

8. Verfahren nach Anspruch 7, wobei das Antikörper-Bindeprotein jenes von Fig. 1, Fig. 2 oder Fig. 3 ist.

9. Verfahren nach einem der Ansprüche 1-8, wobei die Hybridomzellen Rag1 und/oder Rag2 (über)exprimieren.

10. Verfahren nach einem der Ansprüche 1-9, wobei die Antigene von einer Antigen-Bibliothek stammen.

11. Verfahren nach einem der Ansprüche 1-10, wobei die Antigene an einen Träger gebunden sind.

12. Verfahren nach Anspruch 11, wobei der Träger Magnetobeads umfaßt.

13. Verfahren nach einem der Ansprüche 1-10, wobei die Antigene eine Fluoreszenz- oder Biotinmarkierung umfassen.

14. Verfahren nach Anspruch 13, wobei die Fluoreszenzmarkierung FITC, TRITC, Cy3, Cy5, Cy5.5, Cy7 und Phycoerythrin umfaßt.

15. Antikörper-Bindeprotein, wobei das Antikörper-Bindeprotein eine Kombination aus dem Signalpeptid einer Maus-Ig-Kappa-. Kette oder eines Maus-MHC-Klasse I k(k) -Moleküls, einer Antikörper-Bindestelle der Proteine A, G, L oder LG und der Transmembran-Domäne von PDGFR oder CD52 umfaßt.

16. Antikörper-Bindeprotein nach Anspruch 15, wobei das Antikörper-Bindeprotein die Aminosäuresequenz von Fig. 1, Fig. 2 bzw. Fig. 3 umfaßt.

17. DNA, kodierend für das Antikörper-Bindeprotein nach Anspruch 15 oder 16, umfassend:
(a) die DNA eines Antikörper-Bindeproteins der Figuren 1, 2 bzw. 3, oder
(b) eine mit der DNA von (a) über den degenerierten Code verwandte DNA.

18. Expressionsvektor, kodierend für die DNA nach Anspruch 17.

19. Zellen, enthaltend den Expressionsvektor nach Anspruch 18.

## Claims

1. A method of selecting monoclonal antibodies, comprising the fusion of B lymphocytes with myeloma cells to form antibody-producing hybridoma cells, wherein the antibodies are presented on the cell surface of the hybridoma cells by means of an antibody binding protein, and the binding of the antibodies to antigens, wherein the antibody binding proteins are inserted in the hybridoma cells via the myeloma cells or in the hybridoma cells via expression vectors coding therefor.

2. The method according to claim 1, wherein the antibody binding protein comprises a signal peptide, an antibody binding site independent of the antibody specificity and a membrane anchor.

3. The method according to claim 2, wherein the antibody binding protein comprises an Fc binding protein or portions thereof.

4. The method according to claim 2, wherein the antibody binding protein comprises a combination of Fc binding proteins or portions thereof.

5. The method according to claim 3 or 4, wherein the Fc binding protein is CD16, CD32 or CD64.

6. The method according to any of claims 2 to 5, wherein the antibody binding protein comprises an antibody binding domain of proteins A, G, L or LG.

7. The method according to claim 2, wherein the antibody binding protein comprises a combination of the signal peptide of a murine lg kappa chain or a murine MHC-class I k(k) molecule, an antibody binding site of proteins A, G, L or LG and the transmembrane domain of PDGFR or CD52.

8. The method according to claim 7, wherein the antibody binding protein is that of figure 1, figure 2 or figure 3.

9. The method according to any of claims 1 to 8, wherein the hybridoma cells (over)express Rag1 and/or Rag2.

10. The method according to any of claims 1 to 9, wherein the antigens are derived from an antigen library.

11. The method according to any of claims 1 to 10, wherein the antigens are bound to a carrier.

12. The method according to claim 11, wherein the carrier comprises magnetobeads.

13. The method according to any of claims 1 to 10, wherein the antigens comprise fluorescence or biotin labeling.

14. The method according to claim 13, wherein the fluorescence labeling comprises FITC, TRITC, Cy3, Cy5, Cy5.5, Cy7 and phycoerythrin.

15. An antibody binding protein, wherein the antibody binding protein comprises a combination of the signal peptide of a murine Ig kappa chain or a murine MHC-class I k(k) molecule, an antibody binding site of proteins A, G, L or LG and the transmembrane domain of PDGFR or CD52.

16. The antibody binding protein according to claim 15, wherein the antibody binding protein comprises the amino acid sequence of figure 1, figure 2 or figure 3.

17. DNA coding for the antibody binding protein according to claim 15 or 16, comprising:
(a) the DNA of an antibody binding protein of figure 1, 2 or 3, or
(b) a DNA related to the DNA of (a) via the degenerated code.

18. An expression vector, coding for the DNA according to claim 17.

19. Cells containing the expression vector according to claim 18.

## Revendications

1. Procédé de sélection d'anticorps monoclonaux, comprenant la fusion de lymphocytes B avec des cellules de myélome en cellules d'hybridome produisant des anticorps, les anticorps étant présentés à la surface des cellules d'hybridome au moyen d'une protéine de liaison des anticorps, et la liaison des anticorps à des antigènes, les protéines de liaison des anticorps étant insérées par l'intermédiaire des cellules de myélome dans les cellules d'hybridome, ou par l'intermédiaire de vecteurs d'expression les codant dans les cellules d'hybridome.

2. Procédé selon la revendication 1, dans lequel la protéine de liaison des anticorps comprend un peptide signai, un site de liaison des anticorps indépendants de la spécificité de l'anticorps et un ancrage membranaire.

3. Procédé selon la revendication 2, dans lequel la protéine de liaison des anticorps comprend une protéine de liaison Fc ou des parties de celle-ci.

4. Procédé selon la revendication 2 dans lequel, la protéine de liaison des anticorps comprend une combinaison de protéines de liaison Fc ou des parties de celles-ci.

5. Procédé selon la revendication 3 ou 4, dans lequel la protéine de liaison Fc est CD16, CD32 ou CD64.

6. Procédé selon l'une quelconque des revendications 2 à 5 dans lequel, la protéine de liaison des anticorps comprend un domaine de liaison des anticorps de la protéine A, G, L ou LG.

7. Procédé de liaison selon la revendication 2, dans lequel la protéine de liaison des anticorps comprend une combinaison du peptide signal d'une chaîne Kappa d'une Ig de souris ou d'une molécule k(k) d'un MHC de classe I de souris, d'un site de liaison des anticorps de la protéine A, G, L ou LG et du domaine transmembranaire de PDGFR ou de CD52.

8. Procédé selon la revendication 7, dans lequel la protéine de liaison des anticorps est celle de la Fig. 1, de la Fig. 2 ou de la Fig. 3.

9. Procédé selon l'une quelconque des revendications 1 à 8, dans lequel les cellules d'hybridome (sur)expriment Ragl ou Rag2.

10. Procédé selon l'une quelconque des revendications 1 à 9, dans lequel les antigènes proviennent d'une banque d'antigènes.

11. Procédé selon l'une quelconque des revendications 1 à 10, dans lequel les antigènes sont liés à un support.

12. Procédé selon la revendication 11, dans lequel le support contient des billes magnétiques (Magnetobeads).

13. Procédé selon l'une quelconque des revendications 1 à 10, dans lequel les antigènes comprennent un marqueur de fluorescence ou de biotine.

14. Procédé selon la revendication 13, dans lequel le marqueur de fluorescence comprend FITC, TRITC, Cy3, Cy5, Cy5.5, Cy7 et phycoérythrine.

15. Protéine de liaison des anticorps, la protéine de liaison des anticorps comprenant une combinaison du peptide signal d'une chaîne Kappa d'une Ig de souris ou d'une molécule k(k) d'un MHC de classe I de souris, d'un site de liaison des anticorps de la protéine A, G, L ou LG et du domaine transmembranaire de PDGFR ou de CD52.

16. Protéine de liaison des anticorps selon la revendication 15, la protéine de liaison des anticorps comprenant la séquence d'acides aminés de la Fig. 1, de la Fig. 2 respectivement de la Fig. 3.

17. ADN, codant pour la protéine de liaison des anticorps selon la revendication 15 ou 16, comprenant :
(a) l'ADN d'une protéine de liaison des anticorps des figures 1, 2 respectivement 3, ou
(b) un ADN apparenté à l'ADN de (a) par l'intermédiaire du code dégénéré.

18. Vecteur d'expression, codant pour l'ADN selon la revendication 17.

19. Cellules contenant le vecteur d'expression selon la revendication 18.
